Europäisches Patentamt

European Patent Office

Office européen des brevets

(19)

(11) Veröffentlichungsnummer: **0 032 562**
**B1**

(12) **EUROPÄISCHE PATENTSCHRIFT**

(45) Veröffentlichungstag der Patentschrift:
14.03.84

(21) Anmeldenummer: 80107963.3

(22) Anmeldetag: 17.12.80

(51) Int. Cl.³: **A 61 K 9/54**, A 61 K 9/52,
A 61 K 9/24

(54) Neue Dipyridamol-Retardformen und Verfahren zu ihrer Herstellung.

(30) Priorität: 12.01.80 DE 3000979

(43) Veröffentlichungstag der Anmeldung:
29.07.81 Patentblatt 81/30

(45) Bekanntmachung des Hinweises auf die Patenterteilung:
14.03.84 Patentblatt 84/11

(84) Benannte Vertragsstaaten:
AT BE CH DE FR IT LI LU NL SE

(56) Entgegenhaltungen:
AU - A - 64 533
BE - A - 877 706
DE - A - 2 336 218
DE - A - 2 415 490
FR - A - 2 223 047
FR - A - 2 353 285
FR - A - 2 390 959

(73) Patentinhaber: **Dr. Karl Thomae GmbH, Postfach 1755, D-7950 Biberach (Riss) (DE)**

(72) Erfinder: **Gruber, Peter, Dr. Dipl.-Chem., Wetterkreuzstrasse 36, D-7950 Biberach 1 (DE)**
Erfinder: **Brickl, Rolf, Dr. Dipl.-Chem., Erlenweg 37, D-7951 Warthausen 1 (DE)**
Erfinder: **Bozler, Gerhard, Dr. Dipl.-Chem., Alpenstrasse 38, D-7950 Biberach 1 (DE)**
Erfinder: **Stricker, Herbert, Prof. Dr. Dipl.-Chem., Stettinerstrasse 2, D-6507 Ingelheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 0 032 562

Neue Dipyridamol-Retardformen und Verfahren zu ihrer Herstellung

Die Erfindung betrifft mit einem Überzug versehene, Dipyridamol enthaltende sphäroide Teilchen. Diese oral zu verabreichenden Teilchen geben, da sie mit einer Dialysemembran versehen sind, den Wirkstoff im Magen-Darm-Trakt in geregelter Weise retardiert ab.

Bei Dipyridamol (2,6-Bis(diäthanolamino)-4,8-dipiperidino-pyrimido[5,4-d]pyrimidin) handelt es sich um einen seit Jahren bewährten Wirkstoff. Die Art der Erkrankungen, die mit diesem Wirkstoff behandelt werden, erfordert im allgemeinen eine Langzeitbehandlung. Derzeit wird eine Instantform 3−4mal pro Tag verabreicht. Eine Retardform würde gegenüber den derzeitigen Instantformen folgende Vorteile bieten:

— Eine Verringerung der Zahl der Einnahmen pro Tag führt zu besserer Compliance der Patienten, was bei einer Langzeitmedikation von besonderer Bedeutung ist.
— Eine verzögerte Resorption führt zu gleichmäßigeren Blutspiegeln, d. h. Blutspiegelspitzen, die zu Nebenwirkungen führen können, und subtherapeutische Spiegel, wie sie bei Instantformen besonders bei längeren Einnahmeintervallen (während der Nacht) auftreten, werden vermieden, d. h. die Sicherheit, Verträglichkeit und Wirksamkeit des Präparates steigen an.

Aufgrund dieser offensichtlichen Vorteile einer Retardform von Dipyridamol hat es daher nicht an Versuchen zu ihrer Realisierung gefehlt.

Derartige Retardformen können bei Wirkstoffen, die nicht schon per se (z. B. lange biologische Halbwertszeit, langsame Auflösung kristalliner Wirkstoffe) »Retardeigenschaften« aufweisen, beispielsweise dadurch erzielt werden, daß

1. der Wirkstoff zusammen mit Hilfsstoffen so formuliert wird, daß er langsam freigegeben wird, z. B. durch Einbetten in eine sich nicht oder nur langsam auflösende Matrix;
2. der Wirkstoff zusammen mit den Hilfsstoffen zu Tabletten oder Pellets usw. geformt wird, die dann mit einem unlöslichen Überzug versehen werden, der eine langsame Freisetzung des Wirkstoffs zur Folge hat.

Ferner sind Überzugsmittel für feste Arzneimittel bekannt (vgl. deutsche Patentschrift 2 415 490), die aus einem darmlöslichen Cellulosederivat und einem in Verdauungsflüssigkeiten unlöslichen Cellulosederivat bestehen, wobei diese Komponenten in obiger Reihenfolge in einem Mischungsverhältnis von 30 bis 70 Gewichtsprozent zu 70 bis 30 Gewichtsprozent zueinander vorliegen. Bekannt sind desweiteren orale Depot-Arzneiformen mit linearer Wirkstofffreigabe im Gastrointestinaltrakt (vgl. DE-AS 2 336 218) enthaltend sphäroide Arzneistoffteilchen, die mit einer Dialysemembran versehen sind, deren Filmbildner 15 bis 70 Gewichtsprozent eines im pH-Bereich des Magen-Darm-Traktes unlöslichen und enzymatisch nicht abbaubaren Celluloseäthers mit einem Alkoxygruppengehalt von 43 bis 50 Gewichtsprozent und 85 bis 30 Gewichtsprozent einer oder mehrerer nur im alkalischen Bereich des Intestinaltraktes löslicher organischer Cellulose im Molekül enthaltender Verbindungen mit einem Gehalt von 5 bis 40 Gewichtsprozent an freien Carboxylgruppen, wie z. B. Hydroxypropylmethylcellulosephthalat, aufweist.

Im allgemeinen gelten für die Entwicklung einer Retardform folgende Voraussetzungen für einen Wirkstoff:

— gute, pH-unabhängige Löslichkeit im gesamten Magen-Darmtrakt
— keine Änderung der Resorptionsgeschwindigkeit im resorbierfähigen Teil des Magen-Darmtraktes.

Dagegen sind die physikalischen und biochemischen Eigenschaften von Dipyridamol für die Entwicklung einer Retardform völlig ungeeignet:

— Die biologische Halbwertzeit von Dipyridamol ist relativ kurz, d. h. einmal bestehende Blutspiegel sinken rasch ab; ein gleichmäßiger Dipyridamolblutspiegel läßt sich nur erreichen, wenn ständig Wirkstoff resorbiert wird.
— Dipyridamol ist in wäßrigem Milieu nur im Sauren löslich, oberhalb pH 4 ist die Substanz praktisch wasserunlöslich. Dies bedeutet, daß Dipyridamol nur im oberen Gastrointestinaltrakt gelöst und damit auch resorbiert werden kann, während es bei den im Darmbereich auftretenden höheren pH-Werten unlöslich bleibt und nicht resorbiert wird.
— Da die Passagezeit durch den Magen und die oberen Darmbereiche (mit ausreichend saurem pH) relativ kurz ist (ca. 0,5−2 Stunden), ist es somit schwierig, eine Resorption über mehrere Stunden zu erreichen. Außerdem kann die Verweildauer im Magen und in den verschiedenen Darmabschnitten sehr stark variieren, d. h. inter- und intraindividuelle Blutspiegelschwankungen sind naturgemäß bei einer Substanz, deren Löslichkeit vom pH abhängt, dann extrem groß, wenn die Substanzbereitung eine langsame Freigabe, wie sie für Retardformen notwendig ist, aufweist.
— Selbst wenn Dipyridamol gelöst in verschiedene Darmabschnitte gebracht wird, nimmt die Resorptionsgeschwindigkeit vom Duodenum zum Colon hin ab.

Aus diesen Gründen hielt der Fachmann daher die Entwicklung eines wirksamen Dipyridamoldepots für ausgeschlossen. Dies zeigen auch die derzeit bekannten »Depotformen« für Dipyridamol.

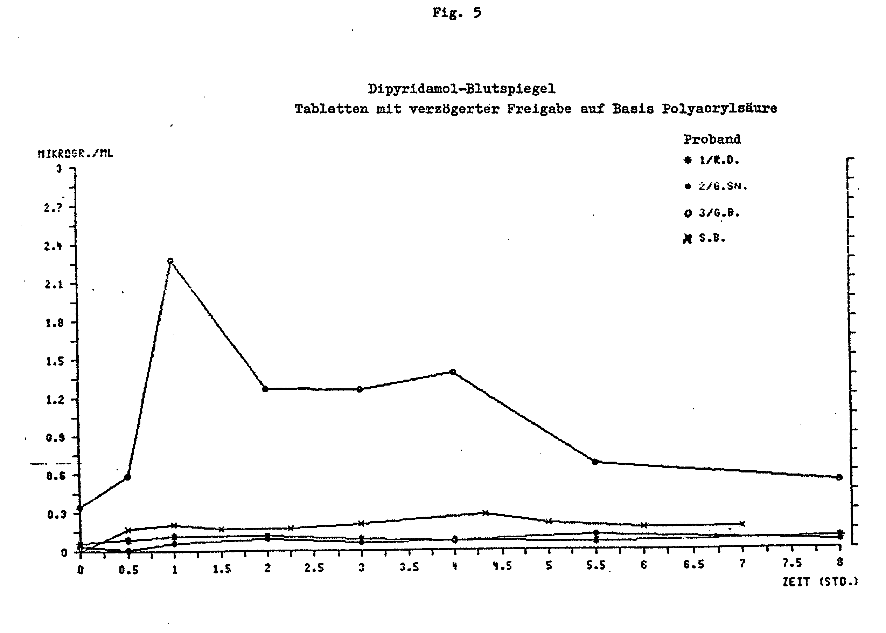

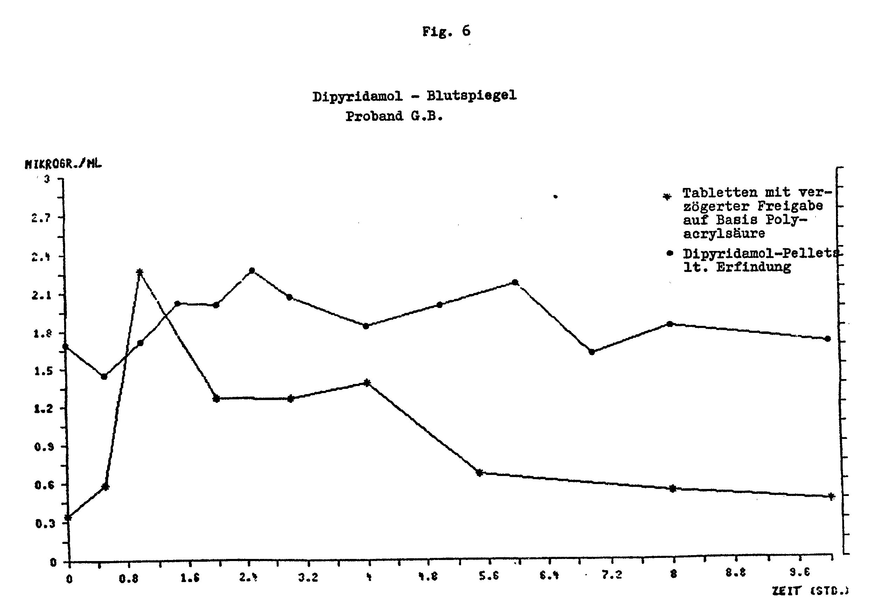

So ist entsprechend der oben in Punkt 1 genannten Möglichkeit eine Retardform bekannt, bei welcher Dipyridamol mit einer unter dem Handelsnamen Carbopol bekannten quellenden Polyacrylsäure zu Matrixtabletten verpreßt wird. Bereits die in vitro-Freigabebestimmung zeigt, daß es sich dabei um eine vollkommen ungeeignete Retardform von Dipyridamol handelt, da bei dieser Form nur solange Dipyridamol in Lösung gehen kann, wie sich die Tablette im sauren Milieu des Magens befindet. Gelangt die Matrixtablette in den Dünndarm, so kommen die Wirkstofffreigabe und damit die Resorption praktisch zum Erliegen. So ergab ein invivo-Versuch (siehe Abb. 5 und 6) mit 4 Versuchspersonen in einer Dosierung von $2 \times 200$ mg Dipyridamol pro Tag bei 3 Versuchspersonen Blutspiegel, die sowohl in bezug auf relative Bioverfügbarkeit als auch Blutspiegelmaxima deutlich unter 10% gegenüber den erfindungsgemäß genannten Formen lag, d. h. als völlige Therapieversager einzustufen sind. Der 4. Proband kam auf einen etwas höheren, trotzdem aber ebenfalls vollkommen ungenügenden Blutspiegel (relative Bioverfügbarkeit ca. 30%). Die völlig ungenügenden und zudem noch stark schwankenden Blutspiegel zeigen, daß von dieser Form keine gesicherte Wirksamkeit erwartet werden kann.

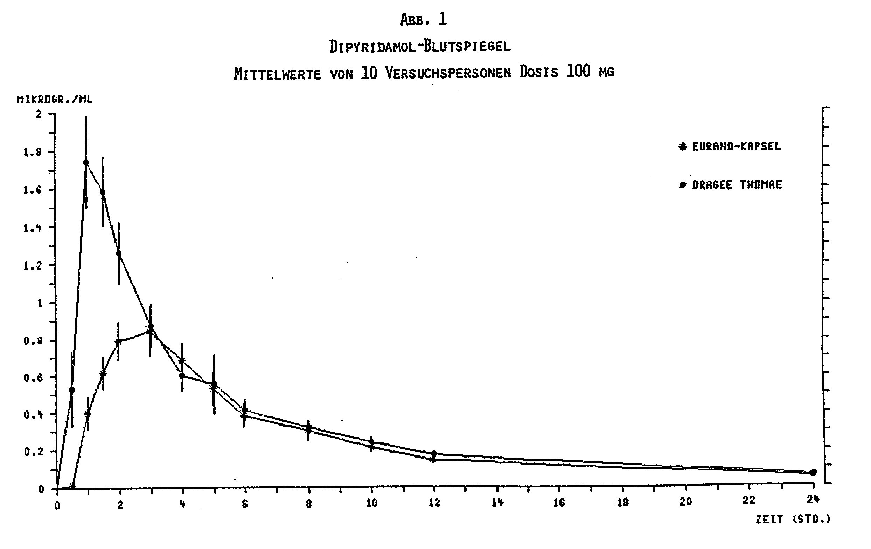

In der französischen Offenlegungsschrift Nr. 7 528 462 (siehe obigen Punkt 2) wird eine an sich moderne Retardform von Dipyridamol in Form von Pellets beschrieben. Der Wirkstoff wird auf indifferente Starterkerne aufgetragen, die anschließend mit einer Retardhülle versehen werden. In dieser Publikation wird beschrieben, daß für die Realisierung eines Dipyridamol-Retard-Präparates Pellets nach dem Eurandverfahren (Überziehen von Pellets mit Hüllen eines Polymeren) geeignet sind. Die bei einem Patienten angegebene Blutspiegelkurve konnte bei einer in vivo-Prüfung an 10 freiwilligen Versuchspersonen, die ebenfalls mit von Eurand hergestellten Dipyridamol-Pellets ausgeführt wurde, in keiner Weise bestätigt werden. Der randomisierte cross-over-Versuch (Retardpellets gegen unverzögerte Dipyridamoldragées siehe Abb. 1) zeigt, daß die Blutspiegel der Dipyridamolretardpellets gegenüber der unverzögerten Handelsform am Anfang sogar deutlich niedriger sind und nicht länger anhalten. Es kann also in keiner Weise von einer Retardform des Dipyridamols gesprochen werden, zumal die relative Bioverfügbarkeit gegenüber den Dragées um ca. die Hälfte verschlechtert ist.

Damit erweisen sich alle bisher bekannten Retardformen von Dipyridamol als vollkommen ungeeignet, obgleich sich mit den verwendeten Technologien bei anderen Wirkstoffen durchaus brauchbare Retardformen herstellen lassen. Aus den bekanntgewordenen Retardformen wird nach dem Eintritt in den Dünndarm infolge des pH-Anstieges kein Dipyridamol mehr aus der Zubereitung herausgelöst, die Resorption des Wirkstoffes kommt zum Erliegen und die Realisierung der gewünschten, langanhaltenden Blutspiegel ist ausgeschlossen.

Die Lösung des Problems, bestehend in der kontinuierlichen, gesteuerten Freisetzung des Dipyridamols im Magen und Darm in resorbierbarer Form, konnte auch nicht der DE-A-2 336 218 entnommen werden, da der dort genannte Säurezusatz der Lösung einer anderen Aufgabe, nämlich zur Verhinderung einer unerwünschten Freisetzung des basischen Wirkstoffes im Magen, diente. Im übrigen ist auch die dort beschriebene Umhüllung, wie bereits vorstehend schon geschildert, nicht geeignet, eine gleichbleibende Freigabe des Wirkstoffes im Magen und Darm zu gewährleisten. Die Verwendung von 15 bis 70 Gew.-% an Ethylcellulose als magen- und darmsaftresistenter Lackanteil hemmt bzw. verhindert die Freisetzung des Dipyridamols in resorbierbarer Form im Darmtrakt. Die Verwendung von darmsaftlöslichen Lacken als Überzüge für Tabletten ist zwar bekannt, sie diente aber immer nur zur Verhinderung der Wirkstofffreisetzung im Magen und nicht zur Verzögerung der Wirkstofffreigabe im Darm.

Es ist nun überraschenderweise gelungen, eine Dipyridamol-Retardform zu finden, die den besonderen Eigenschaften des Dipyridamols voll Rechnung trägt, die oben skizzierten Nachteile bekannter Retardformen für Dipyridamol nicht aufweist und einen langanhaltenden Dipyridamol-Blutspiegel gewährleistet. Dies ist dadurch gelungen, daß eine bisher nicht ausgeführte und nicht auf der Hand liegende Kombination verschiedener pharmazeutischer Technologien zu einem für den Fachmann nicht vorhersehbaren positiven Ergebnis führte.

Folgende Prinzipien wurden dabei angewendet:

1. Die Unlöslichkeit von Dipyridamol bei höherem pH in tieferen Darmabschnitten wird durch Zusatz von sauer wirkenden Stoffen ausgeglichen.
2. Dipyridamol und Säure werden in eine Membran eingeschlossen, die eine rasche Neutralisation der Säure durch den im großen Überschuß vorhandenen Darmsaft verhindert und die sich viel schneller auflösende Säure ausreichend lange zurückhält.
3. Die aus Dipyridamol und Säure gebildeten Teilchen werden mit einer Membran umgeben, die eine speziell für Dipyridamol angepaßte Freigabecharakteristik zeigt, die noch näher beschrieben wird.

Diese Dipyridamol-Retardform besteht im wesentlichen aus einer Reihe sphäroider Teilchen gleicher oder verschiedener Teilchengröße, die sich ihrerseits aus Dipyridamol oder aus kristallisierten Salzen des Dipyridamols und sauren Substanzen, z. B. organischen Genußsäuren, im Verhältnis von mindestens 1 Val Säure auf 1 Mol Dipyridamol zusammensetzen, wobei der Säureanteil aber auch wesentlich höher sein kann, beispielsweise bis zu 30 Val, vorzugsweise jedoch 3 bis 10 Val, und einer die sphäroiden Teilchen umgebenden, eine pH-abhängige Freigabesteuerung ermöglichenden Dialysemembran, bestehend im wesentlichen aus säureunlöslichen, darmsaftlöslichen Lacken, wobei der Hüllenanteil zweckmäßigerweise 3 bis 30 Gewichtsprozent bezogen auf das Gewicht der sphäroiden Teilchen beträgt.

Zur Herstellung der sphäroiden Teilchen wird beispielsweise Dipyridamol mit sauren Substanzen, z. B. organischen Genußsäuren, wie Zitronensäure oder Weinsäure, in einem der oben angegebenen Verhältnisse gemischt und granuliert. Das Granulat kann nach Zusatz von Hilfsstoffen, wie Milchzucker und Magnesiumstearat, zu gewölbten Kernen mit beispielsweise 2 mm Durchmesser verpreßt werden. Die sphäroiden Teilchen können aber auch in Form größerer Kristalle mit sphäroider Gestalt (z. B. bei der Verwendung von kristallisierten Salzen des Dipyridamols), in Form ausgerundeter Granulate oder in Form kleiner Perlen, sogenannter Pellets, vorliegen. Die Herstellung solcher Formen erfolgt nach an sich bekannten Verfahren. Bevorzugt sind kleine Perlen, deren Größe zwischen 0,1 und 3 mm Durchmesser, vorzugsweise zwischen 0,8 und 1,5 mm Durchmesser, liegt.

Unter Säuren versteht man erfindungsgemäß alle toxikologisch unbedenkliche anorganische oder organische Säuren oder in Wasser sauer reagierende Stoffe, wie z. B. Fumarsäure, Apfelsäure, Weinsäure, Zitronensäure, Bernsteinsäure, Ascorbinsäure, saure Salze wie Natrium- bzw. Kaliumhydrogensulfat, Betainhydrochlorid, die Mononatrium- bzw. Kaliumsalze der Weinsäure oder Zitronensäure. Anstelle eines Gemischs aus Dipyridamol und einer Säure gemäß der vorstehenden Definition können auch die sauer in Wasser reagierenden Salze des Dipyridamols, insbesondere solche mit anorganischen Säuren, beispielsweise mit Salzsäure, Schwefelsäure oder Phosphorsäure eingesetzt werden. Als Säuren kommen aber auch Gemische der vorstehend genannten Verbindungen in Frage. Im übrigen eignen sich diese so definierten Säuren auch zum Auftragen, zusammen mit Dipyridamol, auf bereits vorhandene Starterkerne.

Es können als Starterkerne auch andere Säuren bzw. sauer reagierende Stoffe verwendet werden als für die aufzutragende Pulvermischung, d. h. es können auf Starterkerne, die aus den oben genannten organischen Genußsäuren und den sauer reagierenden Stoffen bestehen, jeweils andere Säuren und sauer reagierende Stoffe im Gemisch mit Dipyridamol in der oben beschriebenen Weise aufgetragen werden. Außerdem kann die saure Komponente des auf die Kerne aufzutragenden Gemisches aus mehreren der oben genannten Säuren und sauer reagierenden Stoffe bestehen. Für die Starterkerne sind jene sauren Stoffe besonders geeignet, die annähernd kugelförmige Gestalt aufweisen, z. B. Weinsäure, Zitronensäure, Apfelsäure, Bernsteinsäure, Ascorbinsäure, Na- oder Kaliumhydrogensulfat, Mono-Natrium oder -Kaliumsalze der mehrbasischen Säuren, Betainhydrochlorid.

Das Verhältnis Dipyridamol/saure Substanzen muß so gewählt werden, daß eine vollständige Freisetzung des Dipyridamols gewährleistet ist. Da diese auch vom verwendeten Hüllentyp abhängig ist, werden Einzelheiten später beschrieben.

Die Teilchen bzw. Pellets werden, wie oben bereits erwähnt, nach an sich bekannten Verfahren erzeugt, beispielsweise mit Hilfe von Dragieranlagen nach dem Merumeriza-Verfahren aus Säure- und Dipyridamolpulver unter Zuhilfenahme von Klebstofflösungen. Die Herstellung erfolgt auch beispielsweise unter Benützung eines Pelletiertellers bzw. von Feuchtmischgeräten mit speziellen Rührarmen. Bevorzugt erzeugt man jedoch die Pellets durch Auftragen des Wirkstoffes auf Starterkerne, die entweder aus den üblichen inerten Materialien wie Zucker oder Zuckeralkoholen usw. bestehen oder aber aus den bereits beschriebenen geeigneten Säuren in Gegenwart eines Haftmittels. Die Verwendung von sauren Substanzen hat zwei besondere Vorteile:

1. Die in Retardformen vorzugsweise anzuwendende Dosierung von Dipyridamol liegt bei 150 – 250 mg pro Dosis. Da zweckmäßigerweise die ungefähr gleiche Gewichtsmenge Säure notwendig ist, kann diese Dosis nur dann in eine noch gut schluckbare Kapsel eingebracht werden, wenn Dipyridamol + Säure 90 – 95% des Rohpellets ausmachen. Dies ist bei Verwendung von inerten Startern nicht möglich.

2. Der zentrale Säurekern, der dann von einem Dipyridamol/Säuregemisch umgeben wird, erleichtert die sonst sehr schwierige vollständige Freisetzung von Dipyridamol.

Geeignete Haftmittel sind Klebstofflösungen, wie Stärkekleister Zuckersirup und Lösungen von Gelatine, Guar-Gummi, Celluloseäther (z. B. Methyl-, Äthyl-, Hydroxyäthyl-, Hydroxypropylmethylcellulose) oder Polyvinylpyrrolidon.

Im einzelnen geht man bei einer bevorzugten Verfahrensmethode so vor, daß man ausgerundete

4

Starterkerne von Weinsäure mit einem mittleren Durchmesser von 0,3 – 1 mm, vorzugsweise jedoch von 0,5 – 0,7 mm, in einem geeigneten Kessel gleichmäßig mit einer alkoholischen Polyvinylpyrrolidonlösung besprüht und mit einer Mischung aus 80 Teilen Dipyridamol und 20 Teilen Weinsäure versetzt bis die Kügelchen wieder frei rollen. Nach dem Trocknen wird dieser Vorgang sooft wiederholt, bis der gesamte Wirkstoff aufgetragen ist. Die entstehenden Dipyridamol-Pellets haben eine Größe von 0,9 bis 1,2 mm und bestehen vorzugsweise zu mehr als 95% aus Wirkstoff und Säure im Verhältnis 1,0 zu 1,1. Es ist aber auch möglich, den Wirkstoff in der Klebestofflösung zu lösen bzw. zu suspendieren und diese Lösung oder Suspension gleichmäßig auf die Oberfläche der Starterkerne aufzutragen.

Umfangreiche in-vitro- und in-vivo-Versuche haben gezeigt, daß der Zusammensetzung des Lackes eine ganz wesentliche Bedeutung zukommt. Der auf den Pellets aufgesprühte Lack darf sich im resorptionsfähigen Teil des Gastrointestinaltraktes nicht auflösen, die Hülle muß solange im Intestinaltrakt erhalten bleiben, bis der gesamte Wirkstoff herausdiffundiert ist. Die Hülle muß nämlich die im Kern befindliche Säure solange zurückhalten, bis das darin sich befindende Dipyridamol gänzlich aufgelöst ist. Geht die Hülle vorzeitig in Lösung bzw. wird sie undicht, so dringt der im großen Überschuß vorhandene Darmsaft in das Innere der Pellets bzw. Sphäroide ein und neutralisiert die dort vorhandene Säure. Damit kann wegen der praktischen Unlöslichkeit des Dipyridamols beim pH-Bereich des Darmes kein Wirkstoff mehr in Lösung gehen und resorbiert werden. Die im Inneren des Pellets vorhandene Säure, die sich in der Flüssigkeit auflöst, löst ihrerseits das Dipyridamol auf und schleppt dieses durch die Membran des Retardpellets. Durch die Steigerung der Durchlässigkeit der Umhüllung im Intestinaltrakt wird beim Fortschreiten des Pellets in die tieferen Bezirke des Darmtraktes vermehrt saure Wirkstofflösung abgegeben.

In-vitro-Freigabeversuche mit künstlichem Darmsaft von pH 6,0 – 7,0 zeigen, daß der Wirkstoff Dipyridamol aus den überzogenen Retardformen diffundiert, obwohl er ab pH 4 praktisch unlöslich ist. Offensichtlich wird der Darmsaft beim Eindringen in die Retardform durch die vorhandene Säure umgepuffert. Es herrscht trotz des die Retardform umgebenden Darmsaftes von pH 6,0 bis 7,0 innerhalb der Retardform ein saures Milieu, wodurch das Dipyridamol gelöst und in dieser gelösten Form nach außen diffundieren kann; in den Intestinaltrakt wird somit laufend gelöstes, resorptionsfähiges Dipyridamol abgegeben. Es war überraschend und nicht vorhersehbar, daß aus der Zubereitung ausgetretenes Dipyridamol in dieser Form über längere Zeit resorbierbar bleibt, obgleich z. B. selbst mikronisiertes Dipyridamol, das in den Darmsaft eingebracht wird, nicht resorbiert wird. Ursache dieses unerwarteten Effektes könnte entweder eine auch in vitro zu beobachtende langanhaltende Dipyridamol-Übersättigung des Darmsaftes sein oder das Dipyridamol fällt molekulardispers aus und steht in dieser fein verteilten Form zur Resorption zur Verfügung.

Da gelöstes Dipyridamol unmittelbar nach Verlassen des Magens im obersten Darmabschnitt besonders rasch resorbiert wird, was zu hochschießenden Blutspiegelspitzen führen kann, andererseits in den tieferen Darmabschnitten die Resorptionsgeschwindigkeit deutlich abnimmt, verlangen diese Befunde eine Retardhülle, die den Wirkstoff zunächst verzögert und danach beschleunigt freigibt.

Diese Kriterien erfordern einen neuen, auf die ungewöhnlichen Eigenschaften von Dipyridamol ganz spezifisch angepaßten Dialysemembrantyp, der eine gewisse pH-abhängige Freigabesteuerung ermöglicht. Die Hüllsubstanz besteht aus einem magensaftresistenten aber darmsaftlöslichen Lack, der zum ersten Mal als Dialysemembran eingesetzt wird und diese Funktion auch im alkalischen Bereich erfüllt, obwohl man hätte annehmen müssen, daß sich diese Hülle dort auflöst. Die Zusammensetzung der Hülle wird so gewählt, daß sie im pH-Bereich bis 4,5 eine Freigabeverzögerung und darüber mit steigendem pH eine sich beschleunigende Wirkstofffreisetzung zeigt. Die Verweilzeit von Arzneimitteln und der pH-Wert im Magen und in den verschiedenen Darmabschnitten sind jedoch von Person zu Person und bei einer Person zu verschiedenen Zeiten stark unterschiedlich. Eine zu große pH-Abhängigkeit der Freigabe hätte daher auch große Unterschiede im zeitlichen Verlauf der Blutspiegel zur Folge. Teilt man jedoch die Gesamtdosis auf hunderte von selbständigen, kleinen Retardformen auf, so wird eine statistisch gleichmäßige, weitgehend reproduzierbare Passage dieser Retardform durch den Magen-Darmtrakt gewährleistet. Die Auswirkungen der Unterschiede im pH-Gefälle und der Magen-Darmmotilität bei den einzelnen Patienten auf den Dipyridamol-Blutspiegelverlauf werden dadurch weitgehend ausgeglichen. Die Realisierung des Prinzips einer gewissen pH-abhängigen Freigabesteuerung erfordert daher bei Dipyridamol die Verwendung von sphäroiden Teilchen, wie ausgerundete Granulate oder Pellets. Dies konnte in vivo durch einen Vergleich der Blutspiegel nach Gabe von einigen Kernen (Durchmesser 6 mm) bzw. von Pellets bei gleichem Hülltentyp belegt werden.

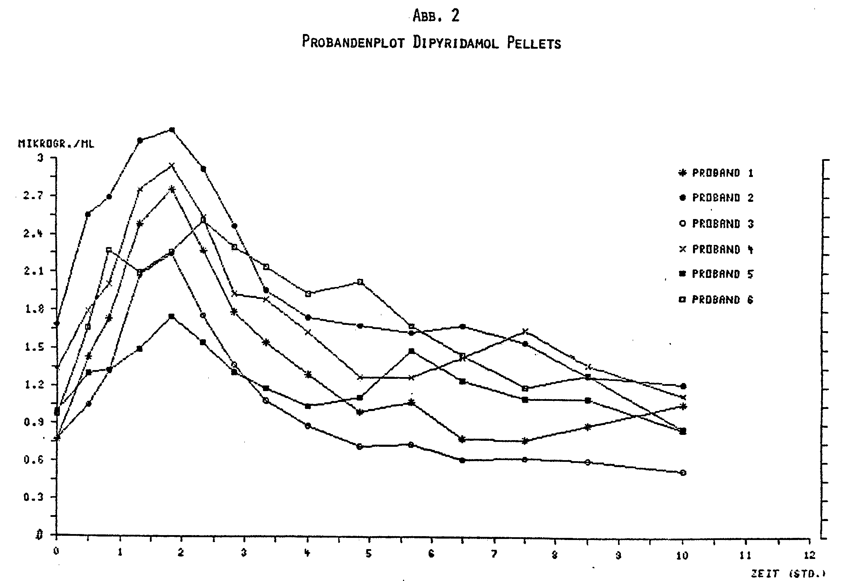

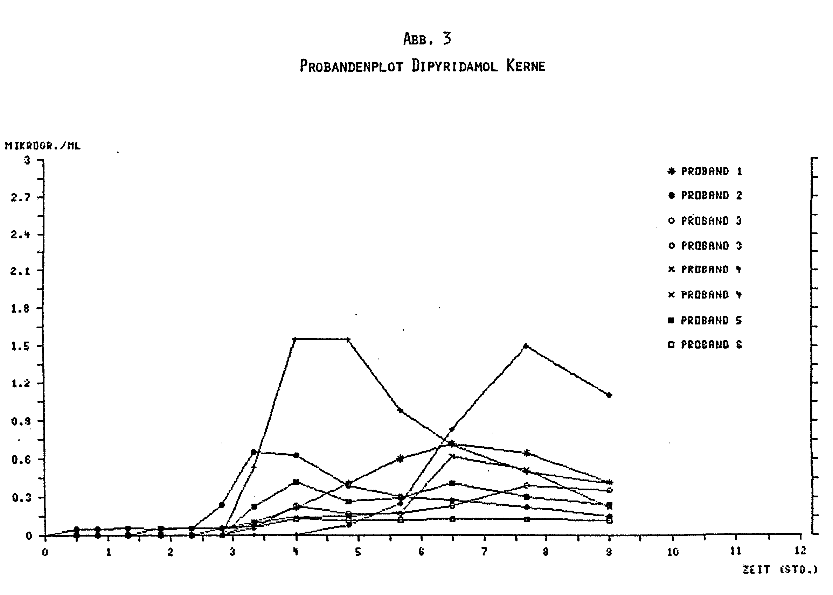

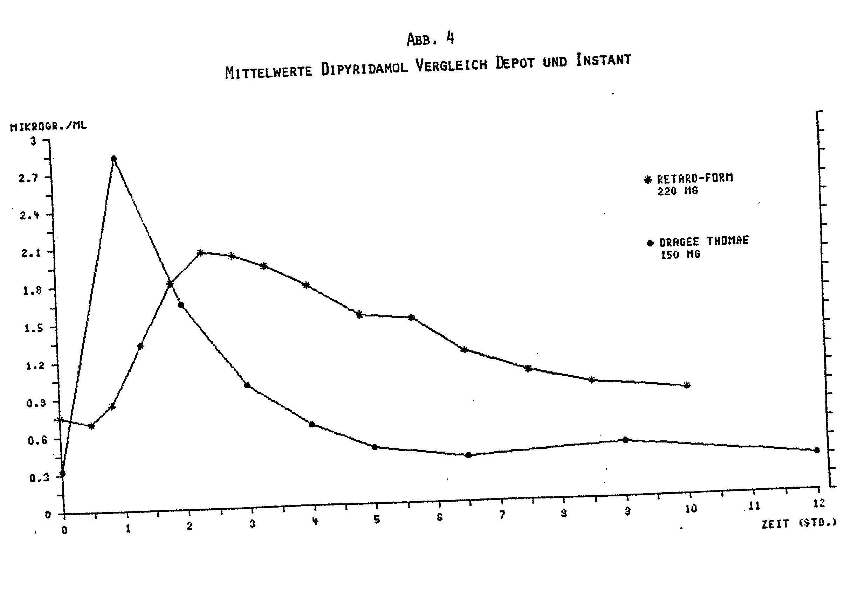

Durch die Abb. 2 und 3 werden die Blutspiegel von jeweils 6 Probanden gezeigt, wobei die Abb. 2 die Dosis auf mehrere hundert Pellets, die in eine Kapsel gefüllt werden, verteilt wird, während bei Abb. 3 6 Kerne in einer Kapsel gegeben wurden. Man sieht, daß die Blutspiegel bei Abb. 2 viel gleichmäßiger aussehen, als bei Abb. 3. Bei Abb. 3 wird außerdem gezeigt, daß auch Blutspiegel von gleichen Probanden, die an verschiedenen Tagen gewonnen wurden, sich stark unterscheiden. Außerdem zeigt der Vergleich mit Abb. 4 (Blutspiegel erfindungsgemäßer Pellets), daß die bei den Pellets offensichtlich wesentlich größere Oberfläche zu bedeutend höheren und länger anhaltenden Blutspiegeln führt.

Die die einzelnen Kügelchen umgebende Dialysemembran besteht weitgehend, bis zu 100 Gewichtsprozent, aus darmsaftlöslichen Lacken. Unter anderem haben sich Zusammensetzungen von 50 – 90 Gewichtsprozent eines Methacrylsäure-Methacrylsäureester-Mischpolymerisats (Säurezahl 180 – 200), bekannt unter dem Handelsnamen Eudragit S, und 50 – 10 Gewichtsprozent Hydroxypropylmethylcellulosephthalat, bekannt unter dem Handelsnamen HP 55, als besonders vorteilhaft erwiesen. Man verwendet zum Besprühen der Sphäroide beispielsweise eine 10- bis 15%ige Lösung beider Komponenten. Obwohl eine derartige Umhüllung nur aus sogenannten darmsaftlöslichen Komponenten besteht, kommt es im resorptionsfähigen Teil des Intestinaltraktes überraschenderweise nicht zur Auflösung der Hülle. Daß derartige Hüllen überhaupt möglich, sogar in diesem Fall besonders geeignet sind, ist aus zwei Gründen überraschend:

1. In der DE-PS 2 415 490 wird ausgeführt, daß zur Erzeugung einer im Darmsaft stabilen Hülle mindestens 30%, vorzugsweise wenigstens 40% in Säure- und Darmsaft unlösliche Hüllenbestandteile notwendig sind.

2. Es war nicht vorhersehbar, daß bei einem derart hohen Anteil an säureunlöslichem Lack überhaupt eine Freigabe zustande kommt, da ja ständig Säure im Inneren gelöst wird und durch die Membran diffundiert.

Als darmsaftlösliche Lackkomponenten kommen z. B. neben den oben genannten auch Celluloseacetatphthalat, Äthylcellulosephthalat, Hydroxypropylmethylcellulosesuccinat, Celluloseacetatsuccinat, Hydroxypropylmethylcellulosehexahydrophthalat, Celluloseacetathexahydrophthalat, Hydroxypropylmethylcellulosetrimellitat oder Methacrylsäure-Methacrylsäureester-Mischpolymerisat (Säurezahl 300 bis 330, auch Eudragit L genannt) für sich oder in Gemischen miteinander in Frage.

Man kann einen gewissen Teil der darmsaftlöslichen Lacke auch durch sowohl im Magen als auch im Darm unlösliche Lacke ersetzen, ohne daß dadurch die optimal wirkende Retardform in ihrer Wirkung allzu große Einbußen erleidet. Als solche Lacke eignen sich neben Äthylcellulose noch Lacksubstanzen auf Acrylat-/Methacrylatbasis, d. h. Lacke, die unter dem Handelsnamen Eudragit retard S und Eudragit retard L bekannt sind. Hierbei können die Säure- und Darmsaft-unlöslichen Lackkomponenten bis zu 50%, vorzugsweise bis zu 30 Gewichtsprozent, betragen.

Wie durch Beispiel 7 gezeigt wird, kann die zunächst abgeschwächte und dann in tieferen Abschnitten des Darmtraktes sich beschleunigende Dipyridamolfreigabe auch durch additiv zusammengesetzte Hüllen erzielt werden. So wird einer Hülle, bestehend aus 14 Gewichtsprozent Äthylcellulose und 86 Gewichtsprozent Hydroxypropylmethylcellulosephthalat, zur weiteren Abschwächung der Freigabe des Dipyridamols im Magen- und oberen Intestinaltrakt noch eine weitere Hülle, bestehend z. B. aus Celluloseacetatphthalat, aufgesprüht.

Wird jedoch der Anteil an darmsaftlöslichen Lackkomponenten zu gering, d. h. sinkt er unter 50 Gewichtsprozent, wird die Freisetzung des Dipyridamols aus den Retard-Pellets bzw. Teilchen unbefriedigend. Dies zeigen sowohl die in-vitro gemessenen Freigabemengen für Dipyridamol (vgl. auch die Beispiele 8 und 9) als auch die in-vivo bestimmten Blutspiegel, die eine schlechte Bioverfügbarkeit (<70% bezogen auf eine Instantform) aufweisen. Ursache dieser schlechten Freisetzung könnte sein, daß die Säure zu rasch durch die Membran diffundiert. Ohne Säure in den Pellets kann aber wegen der Unlöslichkeit des Dipyridamols in einem nichtsauren Milieu kein Wirkstoff mehr gelöst werden und durch die Membran diffundieren. Entweder muß jetzt der Anteil an darmsaftlöslicher Lackkomponente erhöht werden, oder man besprüht zunächst den bei der Herstellung des Granulats oder Pellets eingesetzten Säure-Starterkern mit einer Lösung einer darmsaftlöslichen Lackkomponente, z. B. mit einer Lösung von Celluloseacetatphthalat oder einer Lackkombination aus Eudragit retard S und Eudragit S (z. B. im Verhältnis 1 : 1) und trägt nachträglich das Dipyridamol auf diesen so behandelten retardierten Säure-Starterkern auf.

Die erfindungsgemäß genannten Überzüge bzw. Umhüllungen können die üblichen Hilfsstoffe, wie Weichmacher, Netzmittel und Farbstoffe enthalten. Geeignet sind pharmakologisch unbedenkliche Weichmacher, wie z. B. solche aus der Reihe der Phthalsäure-, Phosphorsäure- oder Zitronensäureglycerinester und Polyäthylenglykole, vorzugsweise wird Glycerintriacetat verwendet.

Das Aufbringen der Dialysenmembran auf die sphäroiden Arzneistoffteilchen erfolgt nach an sich bekannten Methoden. Dies kann in einem rasch rotierenden Kessel oder im Wirbelschichtverfahren durch Aufsprühen der die Dialysemembran bildenden Lacklösung erfolgen.

Der Dosisbereich für den Wirkstoff Dipyridamol liegt zwischen 50 und 500 mg, vorzugsweise jedoch 150 bis 250 mg. Die nach den obenbeschriebenen Verfahren hergestellten sphäroiden Teilchen werden, nachdem sie mit einer Dialysemembran versehen sind, z. B. in Hartgelatine-Kapseln abgefüllt. Dabei ist es möglich, Pellets bzw. Teilchen verschiedener Verzögerungsstufen zu mischen und gegebenenfalls auch unverzögerte Wirkstoffteilchen bzw. -pellets als sogenannte Startdosis hinzuzufügen. Die Dipyridamol-Retardteilchen können aber auch mit anderen pharmazeutischen Hilfsstoffen gemischt und zu Tabletten verpreßt werden. Dies ist bei Teilchen bzw. Pellets mit einem Durchmesser unter 1 mm ohne nennenswerte Beschädigung der Dialysenmembran möglich. Eine solche Tablette zerfällt nach dem Einnehmen in wenigen Sekunden und gibt, wie die Kapseln, die sphäroiden Dipyridamol-Teilchen frei.

**0 032 562**

Die besondere Schwierigkeit in der Auswahl der optimalen Säure, der optimalen Säuremenge, der optimalen Hüllenzusammensetzung und der optimalen Hüllendicke bestand darin, daß sich diese 4 Parameter nicht unabhängig voneinander variieren lassen, sondern sich gegenseitig beeinflussen. Auch aus diesem Grunde war für den Fachmann eine Realisierbarkeit dieses schwierigen Retard-Präparates durch Kombination verschiedener Technologien nicht vorhersehbar.

Durch die Auffindung der erfindungsgemäßen Darreichungsformen wurden folgende Probleme gelöst:

a) Es gelang die Schaffung eines Prinzips, durch welches Dipyridamol überhaupt löslich und unabhängig in seiner Löslichkeit von den pH-Werten des Gastrointestinaltraktes wird.

b) Es wurde eine Diffusionshülle gefunden, die die Säure vor dem vorzeitigen Abpuffern durch den im riesigen Überschuß vorhandenen Darmsaft über Stunden schützt. Die Hülle sorgt für das vollständige Austreten des gelösten Dipyridamols aus den Retardpellets und gleicht die Unterschiede der Dipyridamol-Resorptionsgeschwindigkeiten in den einzelnen Abschnitten des Gastrointestinaltraktes durch eine zunächst verzögerte, dann sich beschleunigende Freigabe bei weiterem Vordringen in die tieferen Abschnitte des Intestinaltraktes aus; die gefundene Hülle bewirkt deshalb anstelle einer linearen Freigabe des Wirkstoffes eine pH-abhängige Freigabe.

c) Durch die Verteilung des Wirkstoffes auf viele hundert Retard-Einzeldosen, z. B. in Form von sphäroiden Teilchen bzw. Pellets oder ausgerundeten Granulaten werden die Auswirkungen der unterschiedlichen Verweilzeiten und des unterschiedlichen pH-Wertes im Gastrointestinaltrakt auf die Blutspiegel durch die statistische Verteilung der Wanderungsgeschwindigkeiten nivelliert.

Aus Abb. 4 geht hervor, wie sich die Blutspiegel bei den erfindungsgemäß beschriebenen Retardformen von einer Instantform unterscheiden. Man sieht, daß trotz der höheren Dosierung von 220 mg bei der Retardform gegenüber 150 mg bei der Instantform niedrigere Blutspiegelmaxima erreicht werden, die aber auf hohem Niveau über mehrere Stunden aufrechterhalten werden. Die Bioverfügbarkeit (berechnet als Fläche unter der Blutspiegelkurve) der neuen Retardformen liegt zwischen 90 und 110% (bezogen auf die Instantformen bei gleicher Dosierung).

Die nachfolgenden Beispiele dienen der weiteren Erläuterung der Erfindung:

Beispiel 1

200 kg ausgerundete Weinsäure-Starterkerne einer Teilchengröße zwischen 0,6 und 0,8 mm werden in einem rotierenden Kessel mit einer 10%igen alkoholischen Polyvinylpyrrolidonlösung (es eignen sich hierzu niedrige Alkohole) gleichmäßig befeuchtet, daraufhin wird solange ein fein pulverisiertes Gemisch aus

| | |
|---|---|
| Dipyridamol | 8 Teile |
| Weinsäure | 2 Teile |

eingestreut, bis die Pellets wieder frei laufen. Nach einer kurzen Trockenphase sprüht man wieder Kleberlösung und trägt danach weiteres Pulver ein. Insgesamt werden auf diese Weise 300 kg der Pulvermischung eingetragen, wozu ca. 150 l Kleberlösung notwendig sind. Die entsprechenden Wirkstoffpellets sind zwischen 0,9 und 1,2 mm groß und enthalten ca. 46% Dipyridamol und 50% Weinsäure. Die Pellets werden nach der letzten Pulverauftragung gut getrocknet.

Beispiel 2

200 kg nicht ausgerundete Zitronensäure-Starterkerne mit einer Teilchengröße von 0,5 bis 0,63 mm werden unter exakt gleichen Bedingungen wie im Beispiel 1 angegeben mit 300 kg einer Pulvermischung aus Dipyridamol und Zitronensäure 8 : 2 auf eine Pelletgröße von 0,8 bis 1,0 mm gebracht.

Als Starterkerne (jeweils 200 kg) wurden außerdem folgende Säuren und sauer reagierende Stoffe verwendet:
Ascorbinsäure, Äpfelsäure, Bernsteinsäure, Natrium- oder Kaliumhydrogensulfat, Betainhydrochlorid, Mononatrium- oder Monokaliumsalze der genannten mehrbasischen organischen Säuren.

Als Säurekomponente der aufzutragenden Mischung aus 8 Teilen Dipyridamol und 2 Teilen Säure werden neben Weinsäure, Zitronensäure ebenfalls die oben genannten Säuren und Salze verwendet. Außerdem können auch Mischungen dieser Stoffe als saure Komponente Verwendung finden.

Das Verhältnis des auf die Kerne aufzutragenden Gemisches aus Dipyridamol und sauer reagierender Komponente kann neben dem oben genannten von 8 : 2 auch folgende Werte aufweisen: 10 : 0, 9 : 1, 7 : 3, 6 : 4, 5 : 5, 4 : 6, 3 : 7, 2 : 8, 1 : 9.

Außerdem können auf 200 kg Starterkerne neben 300 kg der oben genannten Pulvermischungen

7

auch folgende Mengen der oben genannten Zusammensetzungen aufgetragen werden: 100 kg, 200 kg, 400 kg, 500 kg, 600 kg. Die derart hergestellten Wirkstoffpellets sind zwischen 0,7 und 1,5 mm groß. Das Verhältnis Dipyridamol zu saurer Komponente liegt zwischen 3 : 1 (600 kg Dipyridamol auf 200 kg Starterkerne und 1 : 25).

## Beispiel 3

In einem Wirbelschichtgranuliergerät werden 15 kg Dipyridamolpulver mit 17 kg Weinsäurepulver gemischt. Mit Hilfe von 25 kg einer 5%igen Hydroxypropylmethylcelluloselösung (Methylenchlorid/ Isopropanol) wird bei langsamem Sprühen ein Aufbaugranulat hergestellt. 90% des getrockneten, kugelartigen Granulates liegen zwischen 0,6 und 1,0 mm. Der Gehalt an Dipyridamol beträgt ca. 45%.

Anstelle von Weinsäure finden auch Verwendung Zitronensäure, Ascorbinsäure, Fumarsäure, Äpfelsäure, Bernsteinsäure, Mononatrium- und Monokaliumsalze der genannten mehrbasischen Säuren, Natrium- oder Kaliumhydrogensulfat, Betainhydrochlorid.

Der Gehalt an Dipyridamol kann durch Änderung der Zusammensetzung der Mischung auch die folgenden Werte aufweisen: 10%, 20%, 30%, 40%, 50%, 60%, 70%.

## Beispiel 4

19 kg Dipyridamol-Wirkstoffpellets nach Beispiel 1 und 2 werden in einem rasch umlaufenden Dragierkessel mit Schikanen mit einer Lösung von

Methacrylsäure-Methacrylsäureester-
Mischpolymerisat
(Eudragit S®)                                        675 g
Hydroxypropylmethylcellulosephthalat
(HP 55®)                                             675 g

in 8,5 kg Aceton/Isopropanol 1 : 1 besprüht. Als Weichmacher wird 150 g Triacetin zugesetzt.

Die Bestimmung der Wirkstofffreigabe erfolgt nach der Paddle-Methode der USP XX (100 Umdrehungen/Minute). Die Freigabe wird — wenn nicht anders vermerkt — stets unter folgenden Bedingungen getestet.

1 h                          pH 2,0
1 h                          pH 4,5
Rest                         pH 6,0
Die Umpufferung erfolgt mit gesättigter $Na_2HPO_4$-Lösung.

Man erhielt folgende Freigabewerte für Dipyridamol:

1 h                          5,0%
2 h                          23,1%
3 h                          48,0%
4 h                          63,0%
5 h                          75,1%
6 h                          84,0%
7 h                          89,0%

## Beispiel 4a

276 kg Dipyridamol-Wirkstoffpellets nach Beispiel 1 werden in einem rotierenden Kessel diskontinuierlich mit einer Lösung von

Methacrylsäure-Methacrylsäureester-
Mischpolymerisat
(Eudragit S®)                                        19,92 kg
Hydroxypropylmethylcellulosephthalat
(HP 55®)                                             4,08 kg

in 300 kg Aceton/Isopropanol 3 : 7 besprüht. Als Weichmacher wird 8,16 kg Triacetin, also Trennmittel 4,08 kg Talcum zugesetzt.

**0 032 562**

Man erhält folgende Freigabewerte für Dipyridamol:
(Rotatmig basket-Methode, USP XX, 100 Umdrehungen/Minute)
1 Stunde, pH 1,2 (USP-Magensaft)
2 bis 8 Stunden, pH 5,5 (Phosphatpuffer)

| Zeit | Freigesetztes Dipyridamol in % |
|------|-------------------------------|
| 1 h | 5,1 |
| 2 h | 22,9 |
| 3 h | 42,7 |
| 4 h | 54,9 |
| 5 h | 64,7 |
| 6 h | 74,2 |
| 7 h | 82,7 |
| 8 h | 90,6 |

## Beispiel 5

19 kg Dipyridamol-Wirkstoffpellets nach Beispiel 1 werden in einer Wirbelschichtapparatur mit einer Lösung von

| | |
|---|---|
| Äthylcellulose (Äthoxygruppengehalt 48 – 49,5%) | 200 g |
| Copolymerisat aus Acryl- und Methacrylsäureestern (Eudragit retard S®) | 100 g |
| Methacrylsäure-Methacrylsäureester-Mischpolymerisat (Eudragit S®) | 100 g |
| Hydroxypropylmethylcellulosephthalat (HP 55®) | 1200 g |

in 18 kg Aceton/Äthanol 1 : 1 besprüht. Als Weichmacher wird 400 g Triacetin zugesetzt.
Man erhält folgende Freigabewerte für Dipyridamol:

| | |
|---|---|
| 1 h | 2,9% |
| 2 h | 30,0% |
| 3 h | 77,6% |
| 4 h | 88,5% |
| 5 h | 93,0% |

## Beispiel 6

19 kg Dipyridamol-Wirkstoffpellets nach Beispiel 1 werden in einem rasch umlaufenden Dragierkessel mit Schikanen mit einer Lösung von

| | |
|---|---|
| Copolymerisat aus Acryl- und Methacrylsäureestern (Eudragit retard S®) | 200 g |
| Methacrylsäure-Methacrylsäureester-Mischpolymerisat (Eudragit S®) | 200 g |
| Hydroxypropylmethylcellulose-phthalat (HP 55®) | 1200 g |

9

in 14 kg Aceton/Isopropanol 1 : 1 besprüht. Als Weichmacher wird 400 g Triacetin zugesetzt. Man erhält folgende Freigabewerte für Dipyridamol:

| | |
|---|---|
| 1 h | 2,3% |
| 2 h | 14,3% |
| 3 h | 50,1% |
| 4 h | 70,8% |
| 5 h | 79,5% |
| 6 h | 88,2% |
| 7 h | 93,1% |

In analoger Weise wurden auch Hüllen folgender Zusammensetzung auf die Pellets und Granulate, die nach den Beispielen 1 – 3 hergestellt wurden, aufgesprüht:

A) Methacrylsäure-Methacrylsäureester-
Mischpolymerisat
(Eudragit S®)      80 Teile
Triacetin      20 Teile

B) Copolymerisat aus
Acryl- und Methacrylsäureestern
(Eudragit retard S®)      40 Teile
Methacylsäure-Methacrylsäureester-
Mischpolymerisat
(Eudragit S®)      50 Teile
Polyäthylenglykol 6000      10 Teile

C) Copolymerisat aus
Acryl- und Methacrylsäureestern
(Eudragit retard S®)      20 Teile
Methacrylsäure-Methacrylsäureester-
Mischpolymerisat
(Eudragit S®)      70 Teile
Polyäthylenglykol 6000      10 Teile

D) Methacrylsäure-Methacrylsäureester-
Mischpolymerisat
(Eudragit S®)      80 Teile
Methacrylsäure-Methacrylsäureester-
Mischpolymerisat
(Eudragit L®)      10 Teile
Triacetin      10 Teile

E) Celluloseacetatphthalat      60 Teile
Hydroxypropylmethylcellulosephthalat
(HP 55®)      30 Teile
Triacetin      10 Teile

F) Äthylcellulosephthalat      70 Teile
Copolymerisat aus
Acryl- und Methacrylsäureestern
(Eudragit retard S®)      20 Teile
Triacetin      10 Teile

G) Äthylcellulose      10 Teile
Hydroxypropylmethylcellulosesuccinat      75 Teile
Triacetin      15 Teile

H) Hydroxypropylmethylcellulosetrimellitat      35 Teile
Methacrylsäure-Methacrylsäureester-
Mischung-Polymerisat
(Eudragit S®)      60 Teile
Polyäthylenglykol      5 Teile

## Beispiel 7

2,0 kg Dipyridamol-Wirkstoffpellets nach Beispiel 2 werden in einem rasch umlaufenden Dragéekessel mit Schikanen mit einer Lösung von

| | |
|---|---|
| Äthylcellulose (Äthoxygruppengehalt 48—49,5%) | 28 g |
| Hydroxypropylmethylcellulosephthalat (HP 55®) | 172 g |

in 1,8 kg Aceton/Äthanol 1 : 1 besprüht.
Man erhält folgende Freigabewerte für Dipyridamol:

| | |
|---|---|
| 1 h | 8,0% |
| 2 h | 28,1% |
| 3 h | 80,3% |
| 4 h | 90,3% |
| 5 h | 96,5% |

Diese Pellets werden noch mit 80 g Celluloseacetatphthalat, gelöst in 720 ml Aceton, Isopropanol 1 : 4 besprüht.
Die Freigabe reduziert sich auf folgende Werte:

| | |
|---|---|
| 1 h | 4,1% |
| 2 h | 17,5% |
| 3 h | 35,7% |
| 4 h | 53,3% |
| 5 h | 65,6% |
| 6 h | 77,9% |
| 7 h | 86,3% |
| 8 h | 91,2% |

## Beispiel 8

2,0 kg Dipyridamol-Pellets nach Beispiel 1 werden in einem rasch umlaufenden Dragéekessel mit Schikanen mit einer Lösung von

| | |
|---|---|
| Copolymerisat aus Acryl- und Methacrylsäureestern (Eudragit retard S®) | 200 g |
| Copolymerisat aus Acryl- und Methacrylsäureestern (Eudragit retard L®) | 100 g |

in 2,7 kg Aceton/Isopropanol 4 : 6 besprüht. Als Weichmacher wird 30 g Dibutylphthalat zugesetzt.
Man erhält folgende Freigabewerte für Dipyridamol:

| | |
|---|---|
| 1 h | 19,2% |
| 2 h | 38,7% |
| 3 h | 46,7% |
| 4 h | 49,0% |
| 5 h | 52,7% |
| 6 h | 54,1% |
| 7 h | 55,0% |
| 8 h | 55,1% |

## Beispiel 9

2,0 kg Dipyridamol-Pellets nach Beispiel 1 werden in einem rasch umlaufenden Dragéekessel mit Schikanen mit einer Lösung von

| | |
|---|---|
| Copolymerisat aus Acryl- und Methacrylsäureestern (Eudragit retard S®) | 180 g |

Methacrylsäure-Methacrylsäure-
Mischpolymerisat (Eudragit L®)                                                     90 g


in 2,7 kg Aceton/Isopropanol 1 : 1 besprüht. Als Weichmacher wird 30 g Triacetin zugesetzt.
Man erhält folgende Freigabewerte für Dipyridamol:

| | |
|---|---|
| 1 h | 10,4% |
| 2 h | 22,5% |
| 3 h | 35,8% |
| 4 h | 45,1% |
| 5 h | 54,2% |
| 6 h | 61,1% |
| 7 h | 65,0% |
| 8 h | 67,2% |


## Beispiel 10

a) 2,0 kg ausgerundete Weinsäure-Starterkerne einer Teilchengröße von 0,6 – 0,8 mm werden in einem rotierenden Kessel mit einer Lösung von

Methacrylsäure-Methacrylsäureester-
Mischpolymerisat
(Eudragit S®)                                                                        35 g
Copolymerisat aus
Acryl- und Methacrylsäureestern
(Eudragit retard S®)                                                                 35 g

in 620 g Aceton/Isopropanol 1 : 1 besprüht. Als Weichmacher wird 10 g Triacetin zugesetzt.

b) 2,0 kg der überzogenen Weinsäure-Starterkerne nach Beispiel 10a werden in einem rotierenden Kessel mit einer 10%igen alkoholischen Polyvinylpyrrolidonlösung gleichmäßig befeuchtet, daraufhin wird analog Beispiel 1 3 kg einer Pulvermischung aus 8 Teilen Dipyridamol und 2 Teilen Weinsäure aufgetragen. 95% der Pellets liegen zwischen 0,9 und 1,25 mm, der Gehalt an Dipyridamol beträgt 45,6%, an Weinsäure 50,2%.

c) 2,0 kg Dipyridamol-Pellets nach Beispiel 10b werden in einem rotierenden Kessel mit einer Lösung von

Copolymerisat aus
Acryl- und Methacrylsäureestern
(Eudragit retard S®)                                                                 80 g
Methacrylsäure-Methacrylsäureester-
Mischpolymerisat
(Eudragit L®)                                                                       100 g

in 1,8 kg Aceton/Isopropanol 1 : 1 besprüht. Als Weichmacher wird 20 g Triacetin zugesetzt.
Man erhält folgende Freigabewerte für Dipyridamol:

| | |
|---|---|
| 1 h | 8,1% |
| 2 h | 27,3% |
| 3 h | 52,1% |
| 4 h | 64,7% |
| 5 h | 74,0% |
| 6 h | 81,7% |
| 7 h | 88,4% |


## Beispiel 11

2 kg überzogene Dipyridamol-Pellets nach Beispiel 4 mit einem Dipyridamol-Gehalt von 42,0% werden mit 1,5 kg mikrokristalliner Cellulose, 0,4 kg Maisstärke und 0,1 kg Polyvinylpyrrolidon gemischt. Nach Zusatz von 20 g Magnesiumstearat wird noch für 5 Minuten nachgemischt. Aus der Mischung werden unter schwachem Preßdruck 718 mg schwere, oblonge 7 × 13 mm Tabletten gepreßt. Die Tabletten zerfallen in ca. 45 Sekunden; die Dipyridamol-Freigabe hat sich nur unwesentlich beschleunigt.

**0 032 562**

## Patentansprüche für die Vertragsstaaten: BE, CH, DE, FR, IT, LI, LU, NL, SE

1. Neue Dipyridamol-Retardformen, bestehend aus mit einem Überzug versehenen Dipyridamol enthaltenden sphäroiden Teilchen, dadurch gekennzeichnet, daß die Retardform aus sphäroiden Teilchen, die sich ihrerseits aus Dipyridamol und aus Säuren im Verhältnis von mindestens 1 Val Säure auf 1 Mol Dipyridamol zusammensetzen, und eine, die sphäroiden Teilchen umgebende, aus 50 bis 100% säureunlöslichen, darmsaftlöslichen Lacken und entweder 50 bis 0% eines magen- und darmsaftunlöslichen Lackes auf Acrylat-/Methacrylatbasis oder aus 14 bis 0% Äthylcellulose aufgebauten Umhüllung besitzen, besteht.

2. Dipyridamol-Retardformen nach Anspruch 1, dadurch gekennzeichnet, daß der Hüllenanteil 3 bis 30 Gewichtsprozent, bezogen auf das Gewicht der sphäroiden Teilchen, beträgt.

3. Dipyridamol-Retardformen nach Anspruch 1 oder 2, dadurch gekennzeichnet, daß sich die Umhüllung aus 70 bis 100% säureunlöslichen, darmsaftlöslichen Lacken und aus 30 bis 0% eines magen- und darmsaftunlöslichen Lackes auf Acrylat-/Methacrylatbasis zusammensetzt.

4. Dipyridamol-Retardformen nach Anspruch 1, 2 oder 3, dadurch gekennzeichnet, daß das Verhältnis von Säure zu Dipyridamol zwischen 1 Val zu 1 Mol und 30 Val zu 1 Mol beträgt.

5. Dipyridamol-Retardformen nach den Ansprüchen 1 bis 4, dadurch gekennzeichnet, daß die Gesamt-Wirkstoffdosis zwischen 50 und 500 mg liegt.

6. Dipyridamol-Retardformen nach Anspruch 1 bis 5, dadurch gekennzeichnet, daß sie anstelle eines Gemisches aus Säure und Dipyridamol ein in Wasser sauer reagierendes Salz des Dipyridamols und gegebenenfalls zusätzliche Säure enthalten.

7. Dipyridamol-Retardformen nach Anspruch 1 bis 6, dadurch gekennzeichnet, daß als Säuren Fumarsäure, Apfelsäure, Weinsäure, Zitronensäure, Bernsteinsäure, Ascorbinsäure, Natrium- oder Kaliumhydrogensulfat, Betainhydrochlorid, Mononatrium- und Kaliumsalze der Weinsäure oder Zitronensäure enthalten sind.

8. Dipyridamol-Retardformen nach einem der vorhergehenden Ansprüche, dadurch gekennzeichnet, daß die sphäroiden Teilchen in Form ausgerundeter Granulate oder in Form von Pellets vorliegen und einen Durchmesser von 0,1 bis 3 mm aufweisen.

9. Dipyridamol-Retardformen nach einem der vorhergehenden Ansprüche, dadurch gekennzeichnet, daß die darmsaftlöslichen Lacke aus Methacrylsäure-Methacrylsäureester-Mischpolymerisate (Säurezahl 180 bis 200), Hydroxypropylmethylcellulosephthalat, Celluloseacetatphthalat, Äthylcellulosephthalat, Hydroxypropylmethylcellulosesuccinat, Celluloseacetatsuccinat, Hydroxypropylmethylcellulosehexahydrophthalat, Celluloseacetathexahydrophthalat, Hydroxypropylmethylcellulosetrimellitat, Methacrylsäure-Methacrylsäureester-Mischpolymerisat (Säurezahl 300 bis 330) für sich oder in Gemischen miteinander bestehen.

10. Dipyridamol-Retardformen nach Anspruch 9, dadurch gekennzeichnet, daß der darmsaftlösliche Lack aus 90 bis 50 Gewichtsprozent Methacrylsäure-Methacrylsäureester-Mischpolymerisat (Säurezahl 180 bis 200) und 10 bis 50 Gewichtsprozent Hydroxypropylmethylcellulosephthalat besteht.

11. Dipyridamol-Retardform nach einem der vorhergehenden Ansprüche, dadurch gekennzeichnet, daß der Hülle noch eine zweite Hülle, bestehend aus einem darmsaftlöslichen Lack, aufgesprüht wird.

12. Dipyridamol-Retardform nach Anspruch 1 bis 11, dadurch gekennzeichnet, daß ein Starterkern, bestehend aus der Säure, mit einer retardierenden Lackkomponente versehen ist, auf die dann das Dipyridamol, gegebenenfalls im Gemisch mit weiterer Säure, aufgetragen ist.

13. Dipyridamol-Retardformen nach Anspruch 12, dadurch gekennzeichnet, daß für den Starterkern und das aufzutragende Gemisch verschiedene Säuren verwendet werden.

14. Dipyridamol-Retardformen nach einem der vorhergehenden Ansprüche, dadurch gekennzeichnet, daß die sphäroiden Teilchen in Hartgelatine-Kapseln abgefüllt sind.

15. Dipyridamol-Retardformen nach einem der vorhergehenden Ansprüche, dadurch gekennzeichnet, daß die sphäroiden Teilchen, sofern sie einen Durchmesser von bis zu 1,5 mm aufweisen, mit üblichen Hilfsstoffen zu Tabletten verpreßt sind.

16. Verfahren zur Herstellung einer Retardform gemäß den Ansprüchen 1 bis 15, dadurch gekennzeichnet, daß die sphäroiden Teilchen durch Auftragen von Dipyridamol in Pulverform oder als Suspension und, gegebenenfalls, von Säuren auf Starterkerne, bestehend aus inerten Materialien oder einer Säure, in Gegenwart eines Haftmittels gewonnen werden und hernach die die Umhüllung bildende Lacklösung aufgesprüht wird.

17. Verfahren nach Anspruch 16, dadurch gekennzeichnet, daß die sphäroiden Teilchen durch Feucht- oder Trockengranulation aus Dipyridamol und Säuren gebildet werden.

18. Retardform gemäß Anspruch 1 zur Freisetzung einer übersättigten Dipyridamol-Lösung im Darmtrakt.

## Patentansprüche für den Vertragsstaat: AT

1. Verfahren zur Herstellung einer Wirkstoff-Retardform aus sphäroiden Teilchen, bestehend aus Dipyridamol und Säure und einer Umhüllung, wobei Dipyridamol und Säure im Verhältnis von mindestens 1 Val Säure auf 1 Mol Dipyridamol vorliegt und die die sphäroiden Teilchen umgebende Umhüllung aus 50 bis 100% säureunlöslichen darmsaftlöslichen Lacken und entweder aus 50 bis 0% eines magen- und darmsaftunlöslichen Lackes auf Acrylat-/Methacrylatbasis oder aus 14 bis 0% Äthylcellulose besteht, dadurch gekennzeichnet, daß die sphäroiden Teilchen durch Auftragen von Dipyridamol in Pulverform oder als Suspension und, gegebenenfalls, von Säuren auf Starterkerne, bestehend aus inerten Materialien oder einer Säure, in Gegenwart eines Haftmittels hergestellt werden und hernach die die Umhüllung bildende Lacklösung aufgesprüht wird und, falls erwünscht, diese so gewonnenen Retardteilchen anschließend, gegebenenfalls nach Zusatz von üblichen Hilfsmitteln, zu Tabletten verpreßt werden.

2. Verfahren nach Anspruch 1, dadurch gekennzeichnet, daß der Lack bzw. das Lackgemisch solange auf die gebildeten sphäroiden Teilchen aufgesprüht wird, bis der Hüllenanteil 3 bis 30 Gewichtsprozent, bezogen auf das Gewicht der sphäroiden Teilchen, beträgt.

3. Verfahren nach Anspruch 1 und 2, dadurch gekennzeichnet, daß ein Lackgemisch, bestehend aus 70 bis 100% säureunlöslichen, darmsaftlöslichen Lacken und 30 bis 0% eines magen- und darmsaftunlöslichen Lackes auf Acrylat-/Methacrylatbasis, auf die sphäroiden Teilchen aufgesprüht wird.

4. Verfahren nach Anspruch 1, 2 und 3, dadurch gekennzeichnet, daß zwischen Säure und Dipyridamol bei der Herstellung der Retardform ein die Gesamtmengen berücksichtigendes Verhältnis zwischen 1 Val zu 1 Mol und 30 Val zu 1 Mol eingehalten wird.

5. Verfahren gemäß Anspruch 1 und 4, dadurch gekennzeichnet, daß die sphäroiden Teilchen in Form von Pellets auf dem Starterkern aufgebaut werden, wobei solange Dipyridamol gegebenenfalls mit einer Säure aufgetragen wird, bis diese Teilchen einen Durchmesser von 0,5 bis 1,5 mm aufweisen.

6. Verfahren gemäß Anspruch 1, 2 und 3, dadurch gekennzeichnet, daß als darmsaftlösliche Lacke solche aus Methacrylsäure-Methacrylsäureester-Mischpolymerisate (Säurezahl 180 bis 200), Hydroxypropylmethylcellulosephthalat, Celluloseacetatphthalat, Äthylcellulosephthalat, Hydroxypropylmethylcellulosesuccinat, Celluloseacetatsuccinat, Hydroxypropylmethylcellulosehexahydrophthalat, Celluloseacetathexahydrophthalat, Hydroxypropylmethylcellulosetrimellitat, Methacrylsäure-Methacrylsäureester-Mischpolymerisat (Säurezahl 300 bis 330) für sich oder in Gemischen miteinander verwendet werden.

7. Verfahren gemäß Anspruch 6, dadurch gekennzeichnet, daß als darmsaftlöslicher Lack ein solcher verwendet wird, der aus 90 bis 50 Gewichtsprozent Methacrylsäure-Methacrylsäureester-Mischpolymerisat (Säurezahl 180 bis 200) und aus 10 bis 50 Gewichtsprozent Hydroxypropylmethylcellulosephthalat besteht.

8. Verfahren gemäß den Ansprüchen 1 bis 7, dadurch gekennzeichnet, daß auf Starterkerne, bestehend aus einer Säure, eine retardierende Lackkomponente und auf diese das Dipyridamol aufgetragen wird.

9. Verfahren gemäß den Ansprüchen 1 bis 8, dadurch gekennzeichnet, daß die so gewonnenen sphäroiden Teilchen mit einem Durchmesser bis zu 1,5 mm zusammen mit üblichen Hilfsstoffen zu Tabletten verpreßt werden.

10. Verfahren gemäß den Ansprüchen 1, 4 und 5, dadurch gekennzeichnet, daß die sphäroiden Teilchen durch Feucht- oder Trockengranulation aus Dipyridamol und Säuren gebildet werden.

11. Verfahren gemäß Anspruch 1, dadurch gekennzeichnet, daß der Starterkern aus gegebenenfalls vorher ausgerundeten Säurekristallen besteht, die mit einer Dipyridamolsuspension in Gegenwart eines Bindemittels besprüht werden.

## Claims for the contracting states: BE, CH, DE, FR, IT, LI, LU, NL, SE

1. Novel dipyridamole sustained release formulations consisting of spheroidal particles provided with a coating and containing dipyridamole, characterised in that the sustained release formulation consists of spheroidal particles composed of dipyridamole and acid in a ratio of at least 1 equivalent of acid to 1 mol of dipyridamole and a coating which surrounds the spheroidal particles and which is composed of 50 to 100% acid-insoluble lacquers soluble in intestinal juice and either 50 to 0% of an acrylate/methacrylatebased lacquer insoluble in gastric and intestinal juices or 14 to 0% of ethylcellulose.

2. Dipyridamole sustained release formulations according to claim 1, characterised in that the coating constitutes from 3 to 30% by weight of the total weight of the spheroidal particles.

3. Dipyridamole sustained release formulations according to claim 1 or 2, characterised in that the coating is comprised of 70 to 100% acid-insoluble lacquers soluble in intestinal juice and 30 to 0% of an acrylate/methacrylate-based lacquer insoluble in gastric and intestinal juices.

4. Dipyridamole sustained release formulations according to claim 1, 2 or 3 characterised in that the ratio between the acid and dipyridamole is between 1 equivalent to 1 mol and 30 equivalents to 1 mol.

5. Dipyridamole sustained release formulations according to claims 1 to 4, characterised in that the total dose of active substance lies between 50 and 500 mg.

6. Dipyridamole sustained release formulations according to claims 1 to 5 characterised in that they comprise a salt of dipyridamole which is acidic in water and which acts as a mixture of acid and dipyridamole, and optionally an additional acid.

7. Dipyridamole sustained release formulations according to claims 1 to 6, characterised in that they comprise as acid fumaric acid, malic acid, tartaric acid, citric acid, succinic acid, ascorbic acid, sodium or potassium hydrogen sulphate, betaine hydrochloride or the monosodium or potassium salts of tartaric acid or citric acid.

8. Dipyridamol sustained release formulations according to one of the preceding claims, characterised in that the spheroidal particles are present in the form of rounded granulates or in the form of pellets and have a diameter of 0.1 to 3 mm.

9. Dipyridamole sustained release formulations according to one of the preceding claims, characterised in that the lacquers soluble in intestinal juice consist of methacrylic acid/methacrylate copolymers (acid number 180 to 200), hydroxypropylmethylcellulosephthalate, celluloseacetatephthalate, ethylcellulosephthalate, hydroxypropylmethylcellulosesuccinate, celluloseacetatesuccinate, hydroxypropylmethylcellulosehexahydrophthalate, celluloseacetatehexahydrophthalate, hydroxypropylmethylcellulosetrimellitate, methacrylic acid/methacrylat copolymer (acid number 300 to 330) either alone or in admixture.

10. Dipyridamole sustained release formulations according to claim 9, characterised in that the lacquer soluble in intestinal juice consists of 90 to 50% by weight of methacrylic acid/methacrylate copolymer (acid number 180 to 200) and 10 to 50% by weight of hydroxypropylmethylcellulosephthalate.

11. Dipyridamole sustained release formulations according to one of the preceding claims, characterised in that the coating is sprayed with a second coating consisting of a lacquer soluble in intestinal juice.

12. Dipyridamole sustained release formulations according to claims 1 to 11, characterised in that a starter core consisting of the acid is provided with a retarding lacquer component onto which the dipyridamole, optionally mixed with further acid, is then applied.

13. Dipyridamole sustained release formulations according to claim 12, characterised in that different acids are used for the starter core and the applied mixture.

14. Dipyridamole sustained release formulations according to one of the preceding claims, characterised in that the spheroidal particles are filled into hard gelatine capsules.

15. Dipyridamole sustained release formulations according to one of the preceding claims, characterised in that the spheroidal particles have a diameter of up to 1.5 mm and are pressed with conventional excipients into tablets.

16. Process for the preparation of a sustained release formulation according to claims 1 to 15, characterised in that the spheroidal particles are obtained by applying dipyridamole, in the form of a powder or as a suspension, and optionally acid to starter cores consisting of inert material or of an acid in the presence of a bonding agent and the lacquer solution forming the coating is sprayed on thereafter.

17. Process according to claim 16, characterised in that the spheroidal particles are formed by wet or dry granulation of dipyridamole and acid.

18. Sustained release formulation according to claim 1 for the complete release of a supersaturated dipyridamole solution in the intestinal tract.

**Claims for the contracting state: AT**

1. Process for the preparation of active ingredient sustained release formulations consisting of spheroidal particles comprised of dipyridamole and acid and a coating, the dipyridamole and acid being present in a ratio of at least 1 equivalent of acid to 1 mol of dipyridamole and the coating which surrounds the spheroidal particles being comprised of 50 to 100% acid-insoluble lacquers soluble in intestinal juice and either 50 to 0% of an acrylate/methacrylatebased lacquer insoluble in gastric and intestinal juices or 14 to 0% of ethylcellulose, characterised in that the spheroidai particles are prepared by applying dipyridamole, in the form of a powder or as a suspension, and optionaly acid to starter cores consisting of inert material or acid, in the presence of a bonding agent and the lacquer solution forming the coating is sprayed on thereafter and, if desired the sustained release particles thus formed, optionally after addition of conventional excipients, are pressed into tablets.

2. Process according to claim 1, characterised in that the lacquer or lacquer mixture is applied to the formed spheroidal particles whereby the coating constitutes from 3 to 30% by weight of the total weight of the spheroidal particles.

3. Process according to claim 1 and 2 characterised in that a lacquer mixture consisting of 70 to 100% of acid-insoluble lacquers soluble in intestinal juice and 30 to 0% of an acrylate/methacrylatebased lacquer insoluble in gastric and intestinal juices is sprayed onto the spheroidal particles.

4. Process according to claims 1, 2 and 3, characterised in that, in the preparation of the sustained release formulations the total amount of acid and dipyridamole present is maintained in a ratio between 1 equivalent to 1 mol and 30 equivalents to 1 mol.

5. Process according to claims 1 and 4, characterised in that the spheroidal particles are built up in the form of pellets from the starter cores whereby the dipyridamole and optionally an acid is applied until the particle has a diameter of from 0.5 to 1.5 mm.

6. Process according to claims 1, 2 and 3 characterised in that, as lacquer soluble in intestinal juice there is employed methacrylic acid/methacrylate copolymers (acid number 180 to 200), hydroxypropylmethylcellulosephthalate, celluloseacetatephthalate, ethylcellulosephthalate, hydroxypropylmethylcellulosesuccinate, celluloseacetatesuccinate, hydroxypropylmethylcellulosehexahydrophthalate, celluloseacetatehexahydrophthalate, hydroxypropylmethylcellulosetrimellitate, methacrylic acid/methacrylate copolymer (acid number 300 to 330) either alone or in admixture.

7. Process according to claim 6 characterised in that, as lacquer soluble in intestinal juice there is employed 90 to 50% by weight of methacrylic acid/methacrylate copolymer (acid number 180 to 200) and 10 to 50% by weight of hydroxypropylmethylcellulosephthalate.

8. Process according to claims 1 to 7 characterised in that on starter cores consisting of acid there is applied a retarding lacquer component and then dipyridamole.

9. Process according to claims 1 to 8 characterised in that the spheroidal particles are prepared with a diameter of up to 1.5 mm and are pressed together with conventional excipients into tablets.

10. Process according to claims 1, 4 and 5 characterised in that the spheroidal particles are formed by wet or dry granulation of dipyridamole and acid.

11. Process according to claim 1, characterised in that the starter core consists of acid crystals optionally previously rounded which are sprayed with a dipyridamole suspension in the presence of a bonding agent.


**Revendications: Pour les Etats contractants: BE, CH, DE, FR, IT, LI, LU, NL, SE**

1. Nouvelles formes retard du dipyridamole consistant en particules sphéroïdes contenant du dipyridamole, munies d'un enrobage, caractérisées en ce que la forme retard consiste en particules sphéroïdes qui se composent ellesmêmes de dipyridamole et d'acides dans la proportion d'au moins 1 val. d'acide pour 1 mole de dipyridamole, et possédant un enrobage entourant les particules sphéroïdes, constitué de 50 à 100% de laques solubles dans le suc intestinal et insolubles dans les acides et soit de 50 à 0% d'une laque insoluble dans le suc gastrique et dans le suc intestinal, à base d'acrylate/méthacrylate, soit de 14 à 0% d'éthylcellulose.

2. Formes retard du dipyridamole selon la revendication 1, caractérisées en ce que la proportion de l'enrobage est de 3 à 30% en poids par rapport au poids des particules sphéroïdes.

3. Formes retard du dipyridamole selon la revendication 1 ou 2, caractérisées en ce que l'enrobage se compose de 70 à 100% de laques solubles dans le suc intestinal et insolubles dans les acides et de 30 à 0% d'une laque insoluble dans le suc gastrique et dans le suc intestinal, à base d'acrylate/méthacrylate.

4. Formes retard du dipyridamole selon la revendication 1, 2 ou 3, caractérisées en ce que le rapport de l'acide au dipyridamole est compris entre 1 val. pour 1 mole et 30 val. pour 1 mole.

5. Formes retard du dipyridamole selon les revendications 1 à 4, caractérisées en ce que la dose globale de substance active est comprise entre 50 et 500 mg.

6. Formes retard du dipyridamole selon la revendication 1 à 5, caractérisées en ce qu'elles contiennent au lieu d'un mélange d'acide et de dipyridamole un sel du dipyridamole réagissant de façon acide dans l'eau et éventuellement de l'acide supplémentaire.

7. Formes retard du dipyridamole selon la revendication 1 à 6, caractérisées en ce qu'elles contiennent en tant qu'acides de l'acide fumarique, de l'acide malique, de l'acide tartrique, de l'acide citrique, de l'acide succinique, de l'acide ascorbique, du sulfate acide de sodium ou de potassium, du chlorhydrate de bétaïne, des sels monosodés et de potassium de l'acide tartrique pu de l'acide citrique.

8. Formes retard du dipyridamole selon l'une des revendications précédentes, caractérisées en ce que les particules sphéroïdes sont sous forme de granulés arrondis ou sous forme de pastilles et présentent un diamètre de 0,1 à 3 mm.

9. Formes retard du dipyridamole selon l'une des revendications précédentes, caractérisées en ce que les laques solubles dans le suc intestinal sont constituées de polymérisats mixtes d'acide méthacrylique-ester d'acide méthacrylique (indice d'acide 180 à 200), de phtalate d'hydroxypropylméthylcellulose, d'acétophthalate de cellulose, de phtalate d'éthylcellulose, de succinate d'hydroxypropylméthylcellulose, d'acétosuccinate de cellulose, d'hexahydrophtalate d'hydroxypropylméthylcellulose, d'acétohexahydrophtalate de cellulose, de trimellitate d'hydroxypropylméthylcellulose, de polymérisat mixte d'acide méthacrylique-ester d'acide méthacrylique (indice d'acide 300 à 330) en soi ou en mélanges les uns avec les autres.

10. Formes retard du dipyridamole selon la revendication 9, caractérisées en ce que la laque soluble dans le suc intestinal est constituée de 90 à 50% en poids de polymérisat mixte d'acide méthacrylique-ester d'acide méthacrylique (indice d'acide 180 à 200) et de 10 à 50% en poids de phtalate d'hydroxypropylméthylcellulose.

11. Forme retard du dipyridamole selon l'une des revendications précédentes, caractérisée en ce que l'enrobage est en outre muni par pulvérisation d'un deuxième enrobage constitué d'une laque soluble dans le suc intestinal.

12. Forme retard du dipyridamole selon la revendication 1 à 11, caractérisée en ce qu'un noyau de départ constitué de l'acide, est muni d'un composant de laque retardant, sur lequel est apporté ensuite le dipyridamole, éventuellement en mélange avec de l'acide supplémentaire.

13. Formes retard du dipyridamoles selon la revendication 12, caractérisées en ce que pour le noyau de départ et le mélange à faire porter on utilise des acides différents.

14. Formes retard du dipyridamole selon l'une des revendications précédentes, caractérisées en ce que les particules sphéroïdes sont introduites dans des capsules en gélatine dure.

15. Formes retard du dipyridamole selon l'une des revendications précédentes, caractérisées en ce que les particules sphéroïdes, dans la mesure où elle présentent un diamètre allant jusqu'à 1,5 mm, sont pressées en comprimés avec des adjuvants habituels.

16. Procédé pour la préparation d'une forme retard selon les revendications 1 à 15, caractérisé en ce que les particules sphéroïdes sont obtenues par apport de dipyridamole sous forme de poudre ou de suspension, et éventuellement d'acides sur des noyaux de départ constitués de matériaux inertes ou d'un acide, en présence d'un agent d'adhésion et en ce qu'ensuite la solution de laque formant l'enrobage est pulvérisée dessus.

17. Procédé selon la revendication 16, caractérisé en ce que les particules sphéroïdes sont formées de dipyridamole et d'acides, par granulation à l'état humide ou sec.

18. Forme retard selon la revendication 1, pour la libération d'une solution sursaturée de dipyridamole dans le tractus intestinal.


**Revendications: Pour l'Etat contractant: AT**

1. Procédé pour la préparation d'une forme retard de substance active de particules sphéroïdes, constituée de dipyridamole et d'acides et d'un enrobage, le dipyridamole et les acides étant présents dans la proportion d'au moins 1 val. d'acide pour 1 mole de dipyridamole et l'enrobage entourant les particules sphéroïdes étant constitué de 50 à 100% de laques solubles dans le suc intestinal et insolubles dans les acides et soit de 50 à 0% d'une laque insoluble dans le suc gastrique et dans le suc intestinal à base d'acrylate/méthacrylate, soit de 14 à 0% d'éthylcellulose, caractérisé en ce que les particules sphéroïdes sont préparées par apport de dipyridamole sous forme de poudre ou de suspension et, éventuellement, d'acides sur des noyaux de départ constitués de matériaux inertes ou d'un acide, en présence d'un agent d'adhésion et en ce qu'ensuite la solution de laque formant l'enrobage est pulvérisée dessus et en ce que, si on le désire, ces particules retard ainsi obtenues sont pressées ensuite en comprimés, éventuellement après addition d'adjuvants habituels.

2. Procédé selon la revendication 1, caractérisé en ce que la laque ou respectivement le mélange de laques est pulvérisé sur les particules sphéroïdes formées jusqu'à ce que la proportion d'enrobage soit de 3 à 30% en poids, par rapport au poids des particules sphéroïdes.

3. Procédé selon la revendication 1 et 2, caractérisé en ce qu'un mélange de laques constitué de 70 à 100% de laques solubles dans le suc intestinal et insolubles dans les acides et 30 à 0% d'une laque insoluble dans le suc gastrique et dans le suc intestinal, à base d'acrylate/méthacrylate, est pulvérisée sur les particules sphéroïdes.

4. Procédé selon la revendication 1, 2 et 3, caractérisé en ce que l'on maintient entre les acides et le dipyridamole, lors de la préparation de la forme retard, une proportion se rapportant aux quantités totales comprise entre 1 val. pour 1 mole et 30 val. pour 1 mole.

5. Procédé selon la revendication 1 et 4, caractérisé en ce que les particules sphéroïdes sont constituées sous forme de pastilles sur le noyau de départ, le dipyridamole étant apporté, éventuellement avec un acide, jusqu'à ce que les particules présentent un diamètre de 0,5 à 1,5 mm.

6. Procédé selon la revendication 1, 2 et 3, caractérisé en ce que l'on utilise, en tant que laques solubles dans le suc intestinal des laques constituées de polymérisat mixte d'acide méthacrylique-ester d'acide méthacrylique (indice d'acide 180 à 200), de phthalate d'hydroxypropylméthylcellulose, d'acétophthalate de cellulose, de phtalate d'éthylcellulose, de succinate d'hydroxypropylméthylcellulose, d'acétosuccinate de cellulose, d'hexahydrophtalate d'hydroxypropylméthylcellulose, d'acétohexahydrophtalate de cellulose, de trimellitate d'hydroxypropylméthylcellulose, de polymérisat mixte d'acide méthacrylique-ester d'acide méthacrylique (indice d'acide 300 à 330), en soi ou en mélanges les uns avec les autres.

7. Procédé selon la revendication 6, caractérisé en ce que l'on utilise en tant que laque soluble dans le suc intestinal une laque telle que constituée de 90 à 50% en poids de polymérisat mixte d'acide méthacrylique-ester d'acide méthacrylique (indice d'acide 180 à 200) et de 10 à 50% en poids de phtalate d'hydroxypropylméthylcellulose.

8. Procédé selon les revendications 1 à 7, caractérisé en ce que, sur les noyaux de départ constitués d'un acide, on apporte un composant de laque à effet retardant et sur celui-ci le dipyridamole.

9. Procédé selon les revendications 1 à 8, caractérisé en ce que les particules sphéroïdes ainsi obtenues avec un diamètre allant jusqu'à 1,5 mm sont pressées en comprimés, ensemble avec des adjuvants usuels.

10. Procédé selon les revendications 1, 4 et 5, caractérisé en ce que les particules sphéroïdes sont formées par granulation à l'état humide ou à sec, de dipyridamole et d'acides.

11. Procédé selon la revendication 1, caractérisé en ce que le noyau de départ est constitué de cristaux d'acide éventuellement arrondis au préalable sur lesquels est pulvérisée une suspension de dipyridamole en présence d'un liant.

18

ABB. 1

DIPYRIDAMOL-BLUTSPIEGEL

MITTELWERTE VON 10 VERSUCHSPERSONEN DOSIS 100 MG

ABB. 2

PROBANDENPLOT DIPYRIDAMOL PELLETS

ABB. 3

PROBANDENPLOT DIPYRIDAMOL KERNE

ABB. 4

MITTELWERTE DIPYRIDAMOL VERGLEICH DEPOT UND INSTANT

Fig. 5

Dipyridamol-Blutspiegel
Tabletten mit verzögerter Freigabe auf Basis Polyacrylsäure

Proband

\* 1/R.D.

● 2/6.SN.

○ 3/G.B.

✕ S.B.

MIKROGR./ML

ZEIT (STD.)

**Fig. 6**

**Dipyridamol – Blutspiegel**
**Proband G.B.**

Legend:
* Tabletten mit verzögerter Freigabe auf Basis Polyacrylsäure
● Dipyridamol-Pellets lt. Erfindung

Y-axis: MIKROGR./ML
X-axis: ZEIT (STD.)